(19) Europäisches Patentamt
European Patent Office
Office européen des brevets

(11) Veröffentlichungsnummer: **0 041 622**
**B1**

# (12) EUROPÄISCHE PATENTSCHRIFT

(45) Veröffentlichungstag der Patentschrift: **28.03.84**

(51) Int. Cl.³: **C 07 C 69/96,** C 07 C 68/00

(21) Anmeldenummer: **81103576.5**

(22) Anmeldetag: **11.05.81**

(54) **Verfahren zur Herstellung von Carbonaten.**

(30) Priorität: **07.06.80 DE 3021554**

(43) Veröffentlichungstag der Anmeldung: **16.12.81 Patentblatt 81/50**

(45) Bekanntmachung des Hinweises auf die Patenterteilung: **28.03.84 Patentblatt 84/13**

(84) Benannte Vertragsstaaten: **BE CH DE FR GB LI**

(56) Entgegenhaltungen:
**EP - A - 0 013 957**
**EP - A - 0 013 958**

**Houben-Weyl, Methoden der organischen Chemie, Band VIII (1957), Seite 146**

(73) Patentinhaber: **BASF Aktiengesellschaft, Carl-Bosch-Strasse 38, D-6700 Ludwigshafen (DE)**

(72) Erfinder: **Harder, Wolfgang, Dr., Bergwaldstrasse 16, D-6940 Weinheim (DE)**
Erfinder: **Merger, Franz, Dr., Max-Slevogt-Strasse 25, D-6710 Frankenthal (DE)**
Erfinder: **Towae, Friedrich, Dr., Parkstrasse 22, D-6700 Ludwigshafen (DE)**

EP 0 041 622 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Carbonaten

Die Erfindung betrifft ein neues Verfahren zur Herstellung von Carbonaten durch Umsetzung von Carbamidsäureestern mit Alkoholen bei einer Temperatur oberhalb 140° C, wobei man während der Umsetzung den gebildeten Ammoniak abtrennt.

Für die Herstellung von Dialkylcarbonaten werden zur Zeit hauptsächlich folgende Verfahren verwendet: a) Phosgenierung von Alkoholen (Houben-Weyl, „Methoden der org. Chemie", Bd. VIII, S. 105 und 106), b) Alkoholyse von Alkylencarbonaten (DE-AS Nr. 2615665), c) oxidative Carbonylierung von Alkoholen (DE-AS Nr. 2743690, DE-OS Nrn. 2437133 und 2334736).

Allen diesen Verfahren haften schwerwiegende Nachteile an. So erfordert die Herstellung nach Verfahren a eine komplizierte und aufwendige Technik wegen der hohen Toxizität des verwendeten Phosgens. Verfahren b ist betrieblich und wirtschaftlich ungünstig, da die Produktion des Dialkylcarbonates stets eine Koproduktion stöchiometrischer Mengen an Glykol liefert. Verfahren c benötigt eine aufwendige Kohlenmonoxidtechnologie unter Druck. Zusätzlich bereitet das Problem der Rückführbarkeit und Korrosivität der als Katalysatoren eingesetzten Cu-Salze technisch erhebliche Schwierigkeiten.

In der US-PS Nr. 2834799 ist beschrieben, Dialkylcarbonate aus Carbamidsäureestern und Alkohol unter Zusatz mindestens stöchiometrischer Mengen Bortrifluorid herzustellen. Sowohl die Verwendung stöchiometrischer Mengen Bortrifluorid als auch die Beseitigung des bei der Reaktion zwangsweise anfallenden $NH_3-BF_3$-Addukts sind bei diesem Verfahren nachteilig und verhindern eine einfache grosstechnische Herstellung. Die Bildung von Dialkylcarbonaten aus Harnstoff über Carbamidsäureester mit Alkoholen ohne Zusatz stöchiometrischer Mengen Säure wie $BF_3$ erschien bislang unmöglich (Houben-Weyl, „Methoden der org. Chemie", Bd. VIII, S. 105 und 106).

Houben-Weyl (*loc. cit.*, S. 146) zeigte ebenfalls eine Umsetzung von Carbamidsäureestern mit Alkoholen während 8 h bei 190-230° C. Der Esteralkohol ist unterschiedlich von dem als Reaktionspartner dienenden Alkohol, da er weniger Kohlenstoffatome besitzt. Als Endstoff wird der Ester der Carbamidsäure mit dem an Kohlenstoffatomen reicheren Alkohol erhalten; eine deutliche Bildung von Ammoniak wird entsprechend nicht erwähnt. Die Umsetzung entspricht so einer Umsetzung, wobei der kohlenstoffärmere Alkohol während der Umsetzung abdestilliert wird.

Es wurde nun gefunden, dass man Carbonate der Formel I:

$$RO - \underset{\underset{O}{\|}}{C} - OR \qquad (I)$$

worin die Reste R einen aliphatischen, cycloaliphatischen oder araliphatischen Rest bedeuten, durch Umsetzung von Carbamidsäureestern mit Alkohlen vorteilhaft erhält, wenn man Carbamidsäureester der Formel II:

$$H_2N - \underset{\underset{O}{\|}}{C} - OR \qquad (II)$$

worin R die vorgenannte Bedeutung besitzt, mit Alkoholen der Formel III:

$$R - OH \qquad (III)$$

worin R die vorgenannte Bedeutung besitzt, bei einer Temperatur oberhalb 140° C umsetzt, wobei man während der Umsetzung den gebildeten Ammoniak durch Strippen mit unter den Umsetzungsbedingungen inerten Gasen und/oder Dämpfen von inerten Lösungsmitteln abtrennt.

Weiterhin wurde gefunden, dass man die Umsetzung vorteilhaft in Gegenwart von tertiären Aminen oder Amidinen als Katalysatoren durchführt.

Weiterhin wurde gefunden, dass man die Umsetzung vorteilhaft in Gegenwart von Verbindungen der Metalle unter den Elementen der Gruppen Ia, Ib, IIa, IIb, IIIa, IIIb, IVa, IVb, Va, Vb, VIb, VIIb, VIIIb des Periodischen Systems durchführt.

Die Umsetzung kann für den Fall der Verwendung von Carbamidsäuremethylester und Methylalkohol durch die folgenden Formeln wiedergegeben werden:

$$H_2N - \underset{\underset{O}{\|}}{C}OCH_3 + CH_3OH \rightarrow H_3CO - \underset{\underset{O}{\|}}{C} - OCH_3 + NH_3$$

Im Vergleich zu den bekannten Verfahren liefert das Verfahren nach der Erfindung auf einfacherem und wirtschaftlicherem Wege eine grosse Zahl von Carbonaten in guter Ausbeute und Reinheit. Umständliche Abtrennungs-, Entgiftungs- und Aufarbeitungsoperationen, ein Betrieb mit toxischen Stoffen wie Phosgen und Kohlenmonoxid sowie Korrosionsprobleme werden vermieden.

Alle diese vorteilhaften Eigenschaften des erfindungsgemässen Verfahrens sind im Hinblick auf den Stand der Technik überraschend. Auch hätte man wirksame Katalyse für die Carbonatbildung gerade nicht durch tertiäre Amine bzw. Metallverbindungen anstelle der stöchiometrischen Umsetzung mit der starken Lewissäure $BF_3$ erwartet.

Die Ausgangsstoffe II werden mit den Ausgangsstoffen III in stöchiometrischer Menge oder im Überschuss oder Unterschuss umgesetzt, vorteilhaft in einer Menge von 0,9 bis 50 mol, insbesondere von 2 bis 10 mol Ausgangsstoff III/mol Ausgangsstoff II. Bevorzugte Ausgangsstoffe II und III und dementsprechend bevorzugte Endstoffe I sind solche, in deren Formeln die Reste R einen Aralkylrest mit 7 bis 12 Kohlenstoffatomen, einen Alkenylrest mit 2 bis 12, zweckmässig 2 bis 6 Kohlenstoffatomen und bevorzugt einer Doppelbindung, einen Cycloalkylrest mit 5 bis 8 Kohlenstoffatomen oder insbesondere einen Alkylrest mit 1 bis 18, vorteilhaft 1 bis 12, zweckmässig 1 bis 8 Kohlenstoffatomen bezeichnen. Die vorgenannten Reste können noch durch unter den Reaktionsbedingungen inerte Gruppen, z.B. Alkylgruppen oder Alkoxygruppen mit jeweils 1 bis 4 Kohlenstoffatomen, substituiert sein.

Es kommen als Ausgangsstoffe II beispielsweise in Frage: die Ester der Carbamidsäure mit Methanol, Ethanol, Propanol, Isopropanol, sek.-Butanol, Isobutanol, n-Butanol, n-Pentanol, tert.-Amylalkohol, Pentanol-(2),Pentanol-3, Isoamylalkohol, n-Hexanol, Hexanol-2, n-Heptanol, Heptanol-2, 2- und 3-Methylhexanol, 2- und 3-Äthylhexanol, n-Octanol, Octanol-2, n-Nonanol, n-Dekanol, Benzylalkohol, 2-Phenylethanol, Laurylalkohol, Allylalkohol, Äthylenglykolmonomethyläther, Äthylenglykolmonoäthyläther, Äthylenglykolmonobutyläther, Äthylenglykolmonoethoxyäthyläther, Cyclohexanol, Cyclopentanol. Besonders bevorzugt sind Methanol, Ethanol, Propanol, Butanol, Pentanol und Hexanol.

Als Ausgangsstoffe III kommen in Betracht: Methanol, Ethanol, Propanol, Isopropanol, sek.-Butanol, Isobutanol, n-Butanol, n-Pentanol, tert.-Amylalkohol, Pentanol-(2), Pentanol-3, Isoamylalkohol, n-Hexanol, Hexanol-2, n-Heptanol, Heptanol-2, 2- und 3-Methylhexanol, 2- und 3-Äthylhexanol, n-Octanol, Octanol-2, n-Nonanol, n-Dekanol, Benzylalkohol, 2-Phenylethanol, Laurylalkohol, Allylalkohol, Äthylenglykolmonomethyläther, Äthylenglykolmonoäthyläther, Äthylenglykolmonobutyläther, Äthylenglykolmonoethoxyäthyläther, Cyclohexanol, Cyclopentanol. Besonders bevorzugt sind Methanol, Ethanol, Propanol, Butanol, Pentanol und Hexanol.

Die Umsetzung wird bei einer Temperatur oberhalb 140°C, im allgemeinen zwischen 140 und 260°C, vorzugsweise von oberhalb 140 bis 230°C, insbesondere von 160 bis 220°C, drucklos oder unter Druck, vorzugsweise von 0,1 bis 60 bar, kontinuierlich oder diskontinuierlich durchgeführt. Man wählt den Druck zweickmässig so, dass der Alkohol und/oder gegebenenfalls das Lösungsmittel bei der Reaktionstemperatur unter Rücklauf sieden. Zweckmässig dient das Reaktionsgemisch gleichzeitig als Lösungsmedium bzw. Suspensionsmedium. In solchen Fällen ist es mitunter vorteilhaft, einen Überschuss an Ausgangsstoff III schon am Anfang zuzugeben. Gegebenenfalls können unter den Reaktionsbedingungen inerte Lösungsmittel verwendet werden. Vorteilhaft sind Lösungsmittel, in dem der Ammoniak nur gering löslich ist. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin, Mesitylen, Chlorbenzol, o- und m-Dichlorbenzol, o-, m-, p-Chlortoluol, 1,2,4-Trichlorbenzol; Äther, z.B. Äthylpropyläther, Methyl-tert.-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykolmonomethyläther, Tetrahydrofuran, Dioxan, β,β'-Dichlordiäthyläther; aliphatische oder cycloaliphatische Kohlenwasserstoffe, z.B. Heptan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan, Pentan, 2-Methylbutan, Cyclopentan, n-Hexan, Isooctan, n-Decan, n-Undecan, n-Dodecan, n-Hexadecan, n-Octadecan; Ciclosiloxane, z.B. Octamethylcyclotetrasiloxan, Decamethylcyclopentasiloxan, Dodecamethylcyclohexasiloxan, Tetradecamethylcycloheptasiloxan, Hexadecamethylcyclooctasiloxan, Tetrachlorkohlenstoff, Kohlendisulfid; und entsprechende Gemische. Zweckmässig verwendet man das Lösungsmittel in einer Menge von 50 bis 2000, vorteilhaft 100 bis 200 Gew.-%, bezogen auf Ausgangsstoff III.

Man verwendet in einer bevorzugten Ausgangsform als Katalysator ein tertiäres Amin oder ein Amidin, vorteilhaft in einer Menge von 0,0001 bis 1, vorzugsweise von 0,005 bis 0,5, insbesondere 0,01 bis 0,1 Eq Amino- bzw. Amidingruppe im Molekül, bezogen auf 1 mol Ausgangsstoff II. Auch Gemische der genannten Katalysatoren kommen für die Reaktion in Betracht. Das Amin kann in Gestalt von Monoaminen, Diaminen und Polyaminen verwendet werden. Geeignete Katalysatoren sind Trimethylamin, Triäthylamin, Tripropylamin, Triisopropylamin, Tributylamin, Triisobutylamin, Tri-sek.-butylamin, Tribenzylamin, Tricyclohexylamin, Trihexylamin, Dimethylhexylamin, Methyldiisopropylamin, Tetramethyläthylendiamin, Tetramethyltetramethylendiamin, Tetramethylhexamethylendiamin, Tetramethylneopentyldiamin, Dimethyläthylamin, Dimethyllaurylamin, N,N-Dimethylanilin, N,N-Diäthylanilin, N,N-Dimethyltoluidin, Pyridin, α-, β-, γ-Picolin, Chinolin, Isochinolin, Chinazolin, Chinoxalin, p-Dimethylaminopyridin, p-Diäthylaminopyridin, p-Pyrrolidinopyridin, N-Methylimidazol, N-Methylpyrrolidin, 1,4-Diazabicyclo-[2,2,2]-octan, 1,3-Diazabicyclo-[5,4,0]-undecen, 1,5-Diazabicyclo-[4,3,0]-nonen. Ebenfalls kommen auch tertiäre Aminogruppen enthaltende Polymere, z.B. 4-Polyvinylpyridin und Polyvinylimidazol-(N) in Frage. Besonders vorteilhaft sind Triäthylamin, Tripropylamin, Tributylamin, Tetramethyläthylendiamin, Tetramethylpropylendiamin, Tetramethyltetramethylendiamin, Tetramethylpentamethylendiamin, Tetramethylhexamethylendiamin, Tetramethylneopentyldiamin, p-Dimethylaminopyridin, p-Pyrrolidinopyridin, 1,4-Diazabicyclo-[2,2,2]-octan, 1,5-Diazabicyclo-[4,3,0]-nonen, 1,3-Diazabicyclo-[5,4,0]-undecen.

In einer weiteren bevorzugten Ausführungsform werden Verbindungen von den metallischen Elementen der vorgenannten Gruppen als Katalysatoren verwendet. Die Anordnung des Periodischen Systems entspricht D'Ans-Lax, „Taschenbuch für Chemiker und Physiker" (Springer, Berlin, 1967); Bd. 1, S. 53 entsprechend Weast, „Handbook of Chemistry and Physics" (the Chemical Rubber Co., Cleveland, 50. Aufl., S. B 3). Solche Verbindungen sind beispielsweise: Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielsweise können Verbindungen folgender Metalle in Frage kommen: Lithium, Natrium, Kalium, Magnesium, Calcium, Aluminium, Gallium, Zinn, Blei, Wismuth, Antimon, Kupfer, Silber, Gold, Zink, Quecksilber, Cer, Titan, Vanadium, Chrom, Molybdän, Mangan, Eisen, Cobalt und Nickel. Vorzugsweise verwendet man Verbindungen von Lithium, Calcium, Aluminium, Zinn, Wismuth, Antimon, Kupfer, Zink, Titan, Vanadium, Chrom, Molybdän, Mangan, Eisen und Cobalt. Die Katalysatoren können auch in Form ihrer Hydrate oder Ammoniakate zum Einsatz kommen. Die Katalysatoren werden zweckmässigerweise in Mengen von 0,0001 bis 1, vorzugsweise 0,005 bis 0,5, insbesondere 0,01 bis 0,1 Eq Metallkationen, bezogen auf 1 mol Carbamidsäureester, verwendet. Die Metallverbindungen können auch, gebunden an einen Ionenaustauscher, in heterogener Phase eingesetzt werden.

Als Katalysatoren kommen beispielsweise folgende Verbindungen in Betracht: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn(II)acetat, Zinn(II)chlorid, Zinn(IV)chlorid, Bleiacetat, Bleiphosphat, Antimon(III)chlorid, Antimon(V)chlorid, Aluminiumisobutylat, Aluminiumtrichlorid, Wismuth(III)-chlorid, Kupfer(II)acetat, Kupfer(II)sulfat, Kupfer-(II)nitrat, Kupfermolybdat, Silberacetat, Zinkoxid, Zinkchlorid, Zinkacetat, Zinkacetonylacetat, Zinkoctoat, Zinkoxalat, Zinkhexyloat, Zinkbenzoat, Zinkundecylenoat, Cer(IV)oxid, Uranylacetat, Titantetrabutanolat, Titantetrachlorid, Titantetraphenolat, Titannaphthenat, Vanadium(III)-chlorid, Vanadiumacetonylacetat, Chrom(III)-chlorid, Molybdän(VI)oxid, Molybdänacetylacetonat, Wolfram(VI)oxid, Mangan(II)chlorid, Mangan(II)acetat, Mangan(III)acetat, Eisen(II)-acetat, Eisen(III)acetat, Eisenphosphat, Eisenoxalat, Eisen(III)chlorid, Eisen(III)bromid, Cobaltacetat, Cobaltchlorid, Cobaltsulfat, Cobaltnaphthenat, Nickelchlorid, Nickelacetat und Nickelnaphthenat sowie entsprechende Gemische.

Die Umsetzung kann wie folgt durchgeführt werden: Ein Gemisch der Ausgangsstoffe II, III und gegebenenfalls des Katalysators und/oder des Lösungsmittels wird während 1 bis 100 h bei der Reaktionstemperatur gehalten. Gleichzeitig wird das gebildete Ammoniak durch Strippen aus dem Reaktionsgemisch entfernt. Aus dem Gemisch wird dann der Endstoff in üblicher Weise, z.B. durch fraktionierte Destillation, abgetrennt.

Die Abtrennung wird so durchgeführt, dass man während der Umsetzung das Reaktionsgemisch mit unter den Umsetzungsbedingungen inerten Gasen und/oder Dämpfen von inerten Lösungsmitteln strippt. Die Gase und Dämpfe extrahieren den gebildeten Ammoniak aus dem Reaktionsgemisch und dienen somit als Schleppmittel (Strippmittel) der Reinigung des Reaktionsgemischs. Die geschilderte Verfahrensweise wird hier, entsprechend einer Definition in „Introduction to Chemical Engineering" von W.L. Badger und J.T. Banchero (McGraw-Hill Book Comp. Inc. 1955), S. 437 (letzter Absatz), als Strippen bezeichnet. Die inerten Gase und Dämpfe werden in der Regel in einer Menge von 0,01 bis 10 Mol.-%/min, vorzugsweise von 0,01 bis 0,5 Mol.-%/min, bezogen auf 1 mol Ammoniak im Reaktionsgemisch, verwendet. Geeignete Lösungsmittel sind solche, deren Siedepunkt unter 200°C, vorzugsweise zwischen 30 und 140°C liegt. Als inerte Gase sind beispielsweise geeignet: Edelgase wie Argon und Helium, Äthan, Methan, Propan und vorzugsweise Stickstoff und Kohlendioxid.

In einer weiteren bevorzugten Ausführungsform wird in einem ersten Schritt zunächst der Carbamidsäureester II aus Harnstoff und Alkoholen der Formel III:

$$R-OH \qquad (III)$$

worin R die vorgenannte allgemeine und bevorzugte Bedeutung besitzt, vorteilhaft in einem Molverhältnis von 2 bis 50 mol Alkohol IV/mol Harnstoff, während 1 bis 50 h bei einer Temperatur von 120 bis 230°C, drucklos oder unter Druck, kontinuierlich oder diskontinuierlich in Abwesenheit oder Anwesenheit von zweckmässig einem der vorgenannten Lösungsmittel, hergestellt. Dann wird mit dem Reaktionsgemisch ohne Abtrennung des gebildeten Ausgangsstoffs II in einem zweiten Schritt die erfindungsgemässe Umsetzung durchgeführt.

Die nach dem Verfahren der Erfindung herstellbaren Carbonate sind wertvolle Ausgangsstoffe für die Herstellung von Farbstoffen, Pflanzenschutzmitteln und Kunststoffen. Bezüglich der Verwendung wird auf vorgenannte Veröffentlichungen verwiesen.

Die in den folgenden Beispielen aufgeführten Teile bedeuten Gewichtsteile. Sie verhalten sich zu den Volumenteilen wie Kilogramm zu Liter.

*Beispiel 1:*

145 Teile Carbamidsäure-n-hexylester werden mit 204 Teilen Hexanol 20 h auf 180°C erhitzt. Der während der Reaktion gebildete Ammoniak wird unter Verwendung von 1,5 Volumenteilen Stickstoff/Volumenteil Reaktionsgemisch und Stunde als Schleppmittel kontinuierlich abdestilliert. Man erhält (gaschromatographisch geprüft) 27,4 Teile Di-n-hexylcarbonat vom Sp. 121-123°C/4 mbar (96,9% der Theorie, bezogen auf umgesetzten Carbamidsäure-n-hexylester). Der Umsatz beträgt 12,2%.

*Beispiel 2:*

Man verfährt wie in Beispiel 1, gibt jedoch zusätzlich zum Reaktionsgemisch 5 Teile p-Dimethylaminopyridin. Die gaschromatographische Analyse zeigt, dass in derselben Zeit 44,8% Carbamidsäure-n-hexylester umgesetzt sind, wobei 101 Teile Di-n-hexylcarbonat (98,0% der Theorie, bezogen auf umgesetzten Carbamidsäure-n-hexylester) entstanden sind, die durch anschliessende fraktionierende Destillation bei 121-123°C/4 bar abgetrennt werden.

*Beispiel 3:*

6 Teile Harnstoff werden mit 39 Teilen n-Octanol 5 h auf 130°C erhitzt, wobei aus dem Harnstoff 17,3 Teile Carbamidsäure-n-octylester entstehen. Nun werden weitere 39 Teile n-Octanol und 0,5 Teile Tetramethylhexamethylendiamin zu dem Reaktionsgemisch gegeben und 45 h lang auf 195°C erhitzt. Der entstehende Ammoniak wird unter Verwendung von 2 Volumenteilen Stickstoff/Volumenteil Reaktionsgemisch und Stunde kontinuierlich aus der Reaktionslösung abdestilliert. Nach beendeter Reaktion analysiert man die Reaktionsmischung gaschromatographisch sowie durch Dünnschichtchromatographie. Das Reaktionsgemisch enthält 8,8 Teile Carbamidsäure-n-octylester und 13,7 Teile Di-n-octylcarbonat, was einem Umsatz an Carbamidsäure-n-octylester von 49% und einer Ausbeute von Dioctylcarbonat von 97,8%, bezogen auf umgesetzten Carbamidsäure-n-octylester, entspricht.

*Beispiel 4:*

In einer Rührapparatur mit Destillationsaufsatz werden 131 Teile Carbamidsäure-n-pentylester mit 176 Teilen n-Pentanol und 5 Teilen p-Dimethylaminopyridin 19 h lang auf 150°C erhitzt, wobei über ein Druckregelventil im Reaktionsgefäss ein Druck von 2 bis 2,5 bar eingestellt wird. Der während der Reaktion gebildete Ammoniak wird unter Verwendung von 5 Volumenteilen Stickstoff/Volumenteil Reaktionsgemisch und Stunde kontinuierlich abdestilliert. Die gaschromatographische Analyse des abgekühlten Reaktionsgemischs zeigt, dass sich 36% des Carbamidsäure-n-pentylesters umsetzen, wobei 66,2 Teile Di-n-pentylcarbonat (91,0% der Theorie, bezogen auf umgesetzten Carbamidsäurepentylester) vom Kp. 220-221°C (1 bar) entstehen.

*Beispiele 5 bis 9:*

Man verfährt wie in Beispiel 4, setzt jedoch als Katalysatoren andere Amine bzw. Amidine ein. Die Ergebnisse der einzelnen Umsetzungen sind in der folgenden Tabelle 1 aufgeführt.

*Tabelle 1*

| Beispiel | Katalysator | Umsatz (%) | Ausbeute (% der Theorie) |
|---|---|---|---|
| 5 | Diazabicyclo-[2,2,2]-octan | 25 | 92,7 |
| 6 | Tetramethylhexamethylendiamin | 5,5 | 99,3 |
| 7 | N-Methylimidazol | 7,6 | 97,9 |
| 8 | Diazabicyclo-[4,3,0]-nonen | 50,1 | 92,1 |
| 9 | Diazabicyclo-[5,4,0]-undecen | 60,2 | 94,8 |

*Beispiel 10:*

Man verfährt wie in Beispiel 4, ersetzt jedoch n-Pentanol und Carbamidsäure-n-pentylester durch Isoamylalkohol und Carbamidsäureisoamylester und setzt als Katalysator Diazabicyclo-[4,3,0]-nonen zu. Nach beendeter Reaktion ergibt die gaschromatographische Analyse, dass sich 66,9% des Carbamidsäureisoamylesters umsetzten, wobei 120,3 Teile Diisoamylcarbonat vom Sp. 106-108°C/18 mbar (89% der Theorie, bezogen auf umgesetzten Carbamidsäureisoamylester) entstehen.

*Beispiel 11:*

14,5 Teile Carbamidsäure-n-hexylester werden mit 51 Teilen Hexanol 58 h lang zum Sieden (200°C/4-5 bar) erhitzt. Der während der Reaktion gebildete Ammoniak wird unter Verwendung von 5 Volumenteilen Stickstoff/Volumenteil Reaktionsgemisch und Stunde als Schleppmittel kontinuierlich abdestilliert. Nach beendeter Reaktion zeigt die gaschromatographische Analyse der Reaktionslösung, dass sich 18% des Carbamidsäure-n-hexylesters umsetzen, wobei 4 Teile Di-n-hexylcarbonat vom Sp. 122-124°C/4 mbar (96,6% der Theorie, bezogen auf umgesetzten Carbamidsäure-n-hexylester) entstehen.

*Beispiel 12:*

Man verfährt wie in Beispiel 11, gibt jedoch zusätzlich zur Reaktionsmischung 0,5 Teile Zink-(II)acetat. Die gaschromatographische Analyse zeigt, dass sich in derselben Zeit 94% des Carbamidsäure-n-hexylesters umsetzen, wobei 20,3 Teile Di-n-hexylcarbonat (93,9% der Theorie, bezogen auf umgesetzten Carbamidsäure-n-hexylester) vom Kp. 122-124°C entstehen.

*Beispiel 13:*

In einem Rührkessel mit aufgesetzter Destillationsvorrichtung werden 18 Teile Harnstoff mit 45 Teilen Isobutanol während 8 h auf 135°C erhitzt, wobei über ein Druckhalteventil ein Druck von 2 bis 2,5 bar eingestellt wird. Hiernach gibt man weitere 115 Teile Isobutanol und 1 Teil Zink-(II)acetat zu, erhöht die Reaktionstemperatur auf 170°C und kocht weitere 32 h unter Rückfluss, wobei ein Druck von 4 bis 5 bar eingestellt wird; der bei der Reaktion gebildete Ammoniak wird unter Verwendung von 3 Volumenteilen Stickstoff/l Reaktionsgemisch und Stunde als Schleppgas kontinuierlich aus der Reaktionslösung entfernt. Nach beendeter Reaktion lässt man abkühlen und destilliert alle flüchtigen Bestandteile bei einem Druck von 40 mbar ab. Das erhaltene Destillat wird

anschliessend fraktionierend destilliert, wobei 106 Teile Isobutanol und 48,7 Teile Diisobutylcarbonat vom Sp. 83°C/18 mbar (93,3% der Theorie, bezogen auf eingesetzten Harnstoff) erhalten werden. Der Umsatz an Harnstoff ist praktisch quantitativ. Im Destillationsrückstand befinden sich noch 1,1 Teile an rückführbarem Carbamidsäureisobutylester, so dass die Ausbeute an Diisobutylcarbonat, bezogen auf Carbamidsäureisobutylester, 96,3% beträgt.

*Beispiel 14:*

In einem Rührreaktor erhitzt man 117 Teile Carbamidsäurebutylester mit 222 Teilen Butanol und 0,5 Teilen Cobaltacetat, wobei man über ein Druckregelventil im Reaktionsgefäss einen Druck von 9-10 bar einstellt, so dass der Siedepunkt der Reaktionsmischung bei 200°C liegt. Den bei der Reaktion gebildeten Ammoniak destilliert man mit 3 Volumenteilen Stickstoff/l Reaktionsgemisch und Stunde kontinuierlich aus der Reaktionslösung ab. Nach 7 h kühlt man ab und analysiert das Reaktionsgemisch gaschromatographisch. 39,9% des eingesetzten Carbamidsäurebutylesters sind umgesetzt; man erhält 68,3 Teile Dibutylcarbonat vom Sp. 97-98°C/17 mbar (98,4% der Theorie, bezogen auf umgesetzten Carbamidsäurebutylester).

*Beispiele 15 bis 19:*

Man verfährt wie in Beispiel 14, setzt jedoch den Reaktionslösungen andere als Katalysatoren wirkende Verbindungen zu. Die Ergebnisse der einzelnen Umsetzungen sind in der Tabelle 2 aufgeführt.

*Beispiele 20 bis 25:*

Man verfährt wie in Beispiel 14, setzt jedoch anstelle von Butanol und Carbamidsäurebutylester andere Alkohole und Carbamidsäureester ein. Als Katalysatoren werden 0,5 Teile Manganacetat verwendet. Die Ergebnisse der einzelnen Umsetzungen sind in der Tabelle 3 aufgeführt.

*Tabelle 2*

| Beispiel | Temp. (°C) | Druck (bar) | Katalysator | Zeit (h) | Molverhältnis | | Umsatz (%) | Ausbeute (%) |
|---|---|---|---|---|---|---|---|---|
| | | | | | Ausgangsstoff II | Ausgangsstoff III | | |
| 15 | 210 | 10 | Aluminiumtrichlorid | 7 | 1 : | 3 | 34,5 | 92,4 |
| 16 | 210 | 10 | Aluminiumtrichlorid | 7 | 1 : | 4 | 21,6 | 93,5 |
| 17 | 240 | 14 | Tantalpentachlorid | 10 | 1 : | 6 | 92,1 | 73,3 |
| 18 | 210 | 10 | Zinkacetat | 9 | 1 : | 8 | 48,4 | 96,1 |
| 19 | 260 | 22 | Zinkacetat | 7 | 1 : | 6 | 76,1 | 87,0 |

*Tabelle 3*

| Beispiel | Temp. (°C) | Druck (bar) | Ausgangstoffe II, III R | Zeit (h) | Molverhältnis | | Umsatz (%) | Ausbeute (%) |
|---|---|---|---|---|---|---|---|---|
| | | | | | Ausgangsstoff II | Ausgangsstoff III | | |
| 20 | 260 | 52 | $C_2H_5$ | 7 | 1 : | 6 | 59,7 | 79,3 |
| 21 | 200 | 22 | $C_2H_5$ | 7 | 1 : | 6 | 11,6 | 87,6 |
| 22 | 160 | 9 | $C_2H_5$ | 7 | 1 : | 6 | 4,2 | 75,2 |
| 23 | 180 | 19 | $CH_3$ | 7 | 1 : | 6 | 4,8 | 61,5 |
| 24 | 200 | 29 | $CH_3$ | 7 | 1 : | 6 | 15,4 | 57,0 |
| 25 | 160 | 12 | $CH_3$ | 14 | 1 : | 6 | 2,5 | 93,6 |

## Patentansprüche

1. Verfahren zur Herstellung von Carbonaten der Formel (I):

$$RO - \underset{\underset{O}{\|}}{C} - OR \qquad (I)$$

worin die Reste R einen aliphatischen, cycloaliphatischen oder araliphatischen Rest bedeuten, durch Umsetzung von Carbamidsäureestern mit Alkoholen, dadurch gekennzeichnet, dass man Carbamidsäureester der Formel (II):

$$H_2N - \underset{\underset{O}{\|}}{C} - OR \qquad (II)$$

worin R die vorgenannte Bedeutung besitzt, mit Alkoholen der Formel (III):

$$R - OH \qquad (III)$$

worin R die vorgenannte Bedeutung besitzt, bei einer Temperatur oberhalb 140°C umsetzt, wobei man während der Umsetzung den gebildeten Ammoniak durch strippen mit unter den Umsetzungsbedingungen inerten Gasen und/oder Dämpfen von inerten Lösungsmitteln abtrennt.

2. Verfahren nach Anspruch 1, dadurch gekennzeichnet, dass man die Umsetzung in Gegenwart von tertiären Aminen oder Amidinen als Katalysatoren durchführt.

3. Verfahren nach Anspruch 1, dadurch gekennzeichnet, dass man die Umsetzung in Gegenwart von Verbindungen der Metalle unter den Elementen der Gruppen Ia, Ib, IIa, IIb, IIIa, IIIb, IVa, IVb, Va, Vb, VIb, VIIb, VIIIb des Periodischen Systems durchführt.

**Claims**

1. A process for the preparation of a carbonate of the formula (I):

$$RO-\underset{\underset{O}{\|}}{C}-OR \qquad (I)$$

where each of the R's is an aliphatic, cycloaliphatic or araliphatic radical, by reacting a carbamic acid ester with an alcohol, wherein a carbamic acid ester of the formula (II):

$$H_2N-\underset{\underset{O}{\|}}{C}-OR \qquad (II)$$

where R has the above meanings, is reacted with an alcohol of the formula (III):

$$R-OH \qquad (III)$$

where R has the above meanings, at above 140°C, the ammonia formed being removed during the reaction by stripping with gases which are inert under the reaction conditions and/or vapors of inert solvents.

2. A process as claimed in claim 1, wherein the reaction is carried out in the presence of a tertiary amine or amidine as the catalyst.

3. A process as claimed in claim 1, wherein the reaction is carried out in the presence of a compound of a metal chosen from the elements of groups Ia, Ib, IIa, IIb, IIIa, IIIb, IVa, IVb, Va, Vb, VIb, VIIb and VIIIb of the periodic table.

**Revendications**

1. Procédé pour la préparation de carbonates de formule (I):

$$RO-\underset{\underset{O}{\|}}{C}-OR \qquad (I)$$

dans laquelle les symboles R représentent chacun un radical aliphatique, cycloaliphatique ou araliphatique, par réaction d'uréthannes avec des alcools, caractérisé en ce qu'on fait réagir, à une température de plus de 140°C, des uréthannes de formule (II):

$$H_2N-\underset{\underset{O}{\|}}{C}-OR \qquad (II)$$

dans laquelle R a la signification donnée ci-dessus, avec des alcools de formule (III):

$$R-OH \qquad (III)$$

dans laquelle R a la signification donnée ci-dessus, en séparant, au cours de la réaction, l'ammoniac formé, par strippage avec des gaz inertes dans les conditions de la réaction et/ou des vapeurs de solvants inertes.

2. Procédé selon la revendication 1, caractérisé en ce qu'on mène la réaction en présence d'amines tertiaires ou d'amidines servant de catalyseurs.

3. Procédé selon la revendication 1, caractérisé en ce qu'on mène la réaction en présence de composés des métaux choisis parmi les éléments des groupes Ia, Ib, IIa, IIb, IIIa, IIIb, IVa, IVb, Va, Vb, VIb, VIIb, VIIIb du système périodique.